# EUROPEAN PATENT APPLICATION

(11) **EP 1 834 634 A2**
(43) Date of publication of application: **19.09.2007**
(21) Application number: 06004708.1
(22) Date of filing: 08.03.2006
(51) Int. Cl.: A61K 9/16, A61K 9/20, A61K 9/28, A61K 47/10, A61K 47/26, C07D 489/00

(54) **Pharmaceutical multiple-unit composition**

(71) Applicant: Rentschler Pharma GmbH, 88471 Laupheim (DE)
(72) Inventor: Braun, Michael, Dr., 89250 Senden (DE); Neuer, Klaus, 88477 Schwendi (DE); Beckert, Thomas, Dr., 88447 Birkenhard (DE)
(74) Representative: UEXKÜLL & STOLBERG

(57) **Abstract**

The invention relates to pharmaceutical multiple-unit compositions which comprise active ingredient-containing units and protective granules comprising in particular microcrystalline cellulose and polyethylene glycol and which compositions show improved resistance against mechanical damages during compression processes.

## Description

The present invention relates to a pharmaceutical multiple-unit composition and in particular to a composition which includes specific protective granules which protect coated units of active ingredient against damages occurring upon compression and which composition can be in form of an orally disintegrating composition.

In the field of pharmacy, there have been in the past attempts to provide a type of pharmaceutical composition for oral administration which results in improved patient compliance in comparison to conventional solid dosage forms such as capsules and tablets. In particular pediatric and geriatric patients often experience difficulties in swallowing solid dosage forms. Besides, conventional solid dosage forms are not suitable in case the patient has no easy access to water. Thus, orally disintegrating compositions represent an alternative for such patients.

A satisfactory orally disintegrating dosage form needs to meet a number of requirements. Firstly, it has to disintegrate in the mouth spontaneously. However, the active ingredient should not be released from the dosage form in the oral cavity as is the case for sublingual or buccal formulations. Otherwise the active ingredient would be absorbed already in the mouth leading to a different distribution and metabolization of the ingredient in the body compared to a conventional tablet which releases the active ingredient in the gastro-intestinal tract. The consequence would be a difference in relation to bioavailability which would cause significant problems. Moreover, a premature release in the mouth could also lead to problems due to the often unpleasant taste of the active ingredient.

To fulfill all these requirements the formulation for a specific drug or a group of drugs needs to be adapted in particular by a careful selection of the excipients used. However, the excipients may cause a problem as they may lead to formulations which are not bioequivalent to the corresponding conventional dosage forms.

In addition, problems often occur upon processing tableting mixtures including coated pellets by compression processes. The compression often leads to damages of the coating which substantially reduce the function of such coating. For example a gastro-resistant coating or a taste masking coating may substantially be damaged resulting in unsatisfactory tablets.

Several documents describe orally disintegrating pharmaceutical compositions and some of them are dealing with the problem of mechanical damage of coatings as a result of compaction processes.

WO 96/01624 describes multiple unit tableted dosage forms comprising an active substance in the form of an acid labile H⁺ K⁺ -ATPase inhibitor or one of its single enantiomers or an alkaline salt. For the production of tablets, enteric coating layered pellets containing active substance and optionally alkaline substances, are mixed with tablet excipients and compressed into multiple unit tableted dosage forms.

WO 98/53798 discloses a solid preparation which comprises a pharmaceutically active ingredient, one or more water-soluble sugar alcohols selected from the group consisting of sorbitol, maltitol, reduced starch saccharide, xylitol, reduced palatinose and erythritol, and low-substituted hydroxypropyl cellulose having a hydroxypropoxyl group content of 7.0 to 9.9 percent by weight. Tablets are prepared by mixing a granulate having an enteric coating with tablet excipients and subsequent compressing to tablets.

WO 99/59544 relates to an orally disintegrable tablet which comprises fine granules having an average particle diameter of 400 µm or less, which fine granules comprise a composition coated by an enteric coating layer, said composition having 10 weight % or more of an acid-labile physiologically active substance and an additive. The tablets are prepared by mixing the fine granules and the additive and then compressing the mixture.

EP 1 203 580 describes an orally disintegrable tablet which comprises an active ingredient, combined with a coarse powder of a saccharide or a sugar alcohol with a mean particle diameter of 30 µm to 300 µm, a disintegrating agent, and a cellulose compound. Again active ingredient-containing granules are together with additives compressed to give the tablet.

Finally, WO 03/103629 discloses orally disintegrating tablets which disintegrate quickly in the cavity of the mouth and a process for obtaining them. The tablets comprise spray-dried mannitol, active ingredient, microcrystalline cellulose, sodium croscarmellose, and a lubricant, and are prepared by compressing a corresponding powder blend.

However, all these known compositions still suffer from the problem of substantial mechanical damages to the coatings of the granules or pellets when compressed to tablets. Such damages are, however, highly undesirable as the may substantially impair the function of the coatings.

It is, therefore, an object of the present invention to provide compositions wherein such damages are substantially reduced and wherein the function of the coatings employed and in particular of an enteric coating are essentially maintained when subjected to compression. Further, the compositions should reduce segregations effects and therefore should be producible, in particular in form of tablets, with a high uniformity as to mass and content.

This object is surprisingly solved by the pharmaceutical multiple-unit composition according to claims 1 to 28. The invention also relates to the tablet according to claim 29, the protective granules according to claim 30, the process according to claims 31 and 32 and the use according to claims 33 and 34.

The pharmaceutical multiple-unit composition according to the invention comprises
(a) units which comprise at least one active ingredient and at least one coating,
(b) protective granules which comprise
   (i) binder or filler selected from lactose, lactose hydrate, starch, cellulose, sugar alcohol, calcium hydrogenphosphate and mixtures thereof, and
   (ii) additive selected from polyethylene glycol, poloxamer, sugar ester, glycerol fatty acid mono ester, and mixtures thereof, and
(c) optionally at least one tableting excipient,
with the proviso that the active ingredient is not a benzimidazole compound.

The term benzimidazole compound refers to a compound which has at least one benzimidazole moiety and examples of such a compound are lansoprazole, omeprazole, pantoprazole, timoprazole, rabeprazole, eslansoprazole, esomeprazole, salts and stereoisomers thereof.

The units of the composition are in particular beads, particles, granules or pellets and are preferably pellets.

The composition according to the invention preferably comprises 10 to 50 and in particular 20 to 45 % by weight of the units.

The composition according to the invention preferably comprises 10 to 45 and in particular 20 to 40 % by weight of the protective granules.

Moreover, the composition in particular comprises units and protective granules in a weight ratio in the range of 0.4:1 to 5:1 and preferably 0.5:1 to 3:1.

Preferably, the active ingredient is selected from the group of:
Antihistamines, such as dimenhydrinate, diphenhydramine, chlorpheniramine, cetirizine, loratadine and dexchlorpheniramine maleate;
analgesics, such as hydromorphone, aspirin, codeine, morphine, dihydromorphone, and oxycodone;
non-steroid anti-inflammatories, such as naproxen, diclofenac, indomethacin, ibuprofen, and sulindac;
antiemetics, such as metoclopramide;
antiepileptics, such as phenytoin, meprobamate, and nitrazepam;
vasodilators, such as nifedipine, papaverine, diltiazem and nicardipine;
antitussives and expectorants, such as codeine phosphate;
antiasthmatics, such as theophylline;
antacids;
spasmolytics, such as atropine and scopolamine;
antidiabetics, such as insulin;
diuretics, such as ethacrynic acid and bendrofluazide;
agents against low blood pessure, such as propranolol and clonidine;
agents againts high blood pressure, such as clonidine and methyldopa;
bronchodilators, such as albuterol;
steroids, such as testosterone, androgen, estradiol and derivatives thereof, hydrocortisone, triamcinolone,and prednisone;
antibiotics, such as tetracycline;
anthemorrhoidal agents;
hypnotics;
psychotropic agents;
antidepressants, such as amitryptiline;
agents against diarrhoe;
mucolytics;
sedatives;
decongestants;
laxatives; and
vitamins and stimulating agents, including appetite depressants such as phenylpropanolamine;

More preferably the active ingredient is selected from the group of analgesics, antidepressants and non-steroid antiinflammatories.

Most preferred the active ingredient is hydromorphone or amitryptiline.

The term "active ingredient" also includes salts thereof, such as hydromorphone HCl.

The units of the composition comprise at least one coating and such a coating or several coatings are used for their specific functionality. They are usually applied on an inert core or inert seed comprising sugar and starch or microcrystalline cellulose, and such inert cores are commercially available.

A core can also be produced by direct pelletization of the active ingredient together with suitable pharmaceutical excipients, such as binders, fillers, wetting agents, disintegrants and stabilizers, by commonly known methods, such as extrusion spheronization, direct pelletization in tangential pelletizators or high shear mixers. Therefore, the composition according to the invention can also be such that the units comprise an active ingredient-containing core.

It is preferred that the coating of the units is selected from an enteric coating, a controlled-release coating, a taste-masking coating, a separating coating, an intermediate coating, an overcoating, an active ingredient-containing coating or combinations thereof.

The composition of the invention is particularly effective for preventing damage to controlled-release coatings and therefore the units preferably comprise at least one controlled-release coating.

A controlled-release coating is preferably a sustained-release or delayed-release coating.

In a preferred embodiment, the composition comprises active ingredient-containing core, a controlled-release coating and an over-coating.

In a further preferred embodiment, the units comprise an active ingredient-containing coating, a separating coating, a controlled-release coating and an overcoating.

It is preferred that these four coatings are applied on an inert core in the given order to prepare coated units providing controlled drug release, such as pellets. The following description of the composition of theses coatings also apply if they are used singly or in combination with other coatings or if at least some of these coatings are applied on an active ingredient-containing core.

Firstly, the inert cores are usually provided with the active ingredient-containing coating. This first active ingredient-containing coating usually comprises, apart from the active ingredient, at least one additional component selected from the group including:
fillers such as sugar alcohols, preferably mannitol or sorbitol, monosaccharides, preferably glucose, disaccharides, preferably lactose or sucrose, and/or polysaccharides, preferably starch derivatives, such as maize starch, cellulose derivatives, such as microcrystalline cellulose;
stabilizers such as organic and inorganic acids and bases, e.g. magnesium oxide, magnesium carbonate, magnesium phosphate, natural clays, calcium carbonate, calcium oxide, alkali phosphates, carbonates, amine derivates, such as meglumine; and tartic acid, ascorbic acid, and citric acid;
binders such as hydroxypropyl methylcellulose, hydroxypropyl cellulose, hydroxyethyl cellulose, methylcellulose, polyvinylpyrrolidone (povidone); and
wetting agents such as sodium lauryl sulphate, poloxamer, and sorbitan derivatives.

In a preferred embodiment, the active ingredient-containing coating or the active ingredient-containing core comprises hydroxypropyl methylcellulose.

Optionally a separating coating can subsequently be applied between the active ingredient-containing coating and the following controlled-release coating. Such a separating coating is preferably comprising a soluble or swellable polymer and antitacking agent.

The controlled-release coating is subsequently applied as third coating. The controlled-release coating usually comprises at least one of:
acid insoluble polymer preferably selected from methacrylic acid copolymers (such as Eudragit L and S types and Eudragit NE), cellulose acetate phthalate, hydroxypropyl methylcellulose phthalate, hydroxypropyl methylcellulose acetate succinate, and cellulose acetate trimellitate;
hydrophobic polymer selected from alkyl cellulose, acrylic polymer, shellac, zein, polyvinyl acetate, hydrogenated castor oil, and hydrogenated vegetable oil;
plasticizer such as triethyl citrate, acetyl triethyl citrate, dibutyl sebacate, oleic acid, propylene glycol, polyethylene glycol or a combination thereof;
antitacking agent such us talc, or glyceryl monostearate; and
pigment such as titanium dioxide, iron oxides or a mixture thereof.

Preferably the controlled-release coating comprises polyvinyl acetate polymer.

The fourth coating usually is the overcoating. The overcoating preferably comprises:
water soluble binder selected from methylcellulose, hydroxypropyl methylcellulose, hydroxypropyl cellulose, hydroxyethyl cellulose, polyvinyl pyrrolidone, polyethylene glycol and mixtures thereof; and/or
water insoluble substance selected from microcrystalline cellulose, starch and mixtures thereof.

Optionally the overcoating also includes other components selected from the group of antitacking agents, plasticizers and fillers.

The overcoating is generally able to increase the roughness of the surface of the units, such as pellets, and it is assumed that it therefore facilitates the mixing of the units with the protective granules and the tableting excipients.

It has surprisingly been found out that the overcoating is particularly effective in also preventing damages to an underlying coating, such as a controlled-release coating, during the compression to tablets if the overcoating comprises hydroxypropyl methylcellulose and microcrystalline cellulose. Consequently, such an overcoating is particularly preferred.

It is very surprising that a combination of the units with the specific protective granules according to the invention is able to effectively contribute to the reducing of mechanical damages which are caused by the compression forces of a subsequent compaction to tablets. This is impressively shown by the fact that for example the dissolution profile of such a combination including units, namely pellets, having a controlled-release coating is only slightly changed in comparison to the uncompressed pellets. It is assumed that the specific protective granules of the invention are mainly responsible for this advantageous behaviour.

In addition, the combination of the units with the protective granules also leads to a reducing of segregation effects and hence results in a high uniformity of prepared tablets in terms of mass and content of the active ingredient.

The protective granules comprise
(i) binder or filler selected from lactose, lactose hydrate, starch, cellulose, sugar alcohol, calcium hydrogenphosphate and mixtures thereof, and
(ii) additive selected from polyethylene glycol poloxamer, sugar ester, glycerol fatty acid mono ester, and mixtures thereof.

The lactose useful as binder can be any form of lactose such as lactose monohydrate, agglomerated lactose, or spray dried lactose. The starch can be pregelatinized starch or maize starch. The cellulose can be powdered cellulose, microcrystalline cellulose, or silicified microcrystalline cellulose. The preferred sugar alcohol is mannitol or sorbitol. Mixtures are also operable including coprocessed binders such as the material available under the tradename Cellactose^{®}. The preferred binder is microcrystalline cellulose, which is for example available as microcrystalline cellulose 101.

The additive included in the granules has preferably wax-like properties. Polyethylene glycol is a preferred additive and it is in particular a polyethylene glycol H(OCH₂-CH₂)ₙOH wherein n is in the range of 100 to 250 and preferably 150 to 210. A mixture of such preferred polyethylene glycols is commercially available under the name PEG 6000.

A preferred poloxamer HO(CH₂CH₂O)ₐ(CH(CH₃)CH₂OH)_{b}(CH₂CH₂O)_{c} H is type 188 or type 407.

A preferred sugar ester is sucrose stearate. Particularly preferred is type S370F.

A preferred glycerol fatty acid mono ester is glycerol monostearate.

It is particularly preferred that the protective granules comprise microcrystalline cellulose and polyethylene glycol(s) and in particular consist of microcrystalline cellulose and polyethylene glycol(s).

It is also preferred that the protective granules comprise 50 to 95 and preferably 70 to 85 % by weight of the binder or filler (i).

It is further preferred that the protective granules comprise 5 to 50 and preferably 15 to 30 % by weight of the additive (ii).

Moreover, it is preferred that the protective granules comprise binder or filler (i) and additive (ii) in a weight ratio in the range of 1:1 to 20:1 and preferably 2:1 to 5:1.

In a further preferred embodiment, the average particle size of the protective granules determined by sieve or image analysis does not differ by more than 50 % from the average particle size of the units. This further reduces segregation effects occurring during the mixing of units (a), protective granules (b) and optional tableting excipients (c) or their compressing.

The composition optionally comprises also at least one tableting excipient (c). Preferably at least one tableting excipient is present in the composition.

The tableting excipient is preferably at least one disintegrant which is in particular selected from the group of starch, starch derivatives, such as sodium starch glycolate and pregelatinized starch, low-substituted hydroxypropyl cellulose, cross-linked polyvinyl pyrrolidone (crospovidone), polacrilin potassium, and croscarmelose sodium. The disintegrant is preferably cross-linked polyvinyl pyrrolidone.

In a preferred embodiment, the composition comprises 1 to 10 % by weight of disintegrant based on the weight of the composition.

Further, possible tableting excipients (c) include fillers or binders, lubricants, glidants, sweeteners, flavouring agents, and organic acids.

Examples of preferred fillers or binders are sugar alcohols, e.g. mannitol, lactose, microcrystalline cellulose and mixtures thereof.

Suitable lubricants are magnesium stearate, magnesium lauryl sulfate, sodium stearyl fumarate, hydrogenated vegetable oil, fatty acid, and stearic acid.

A suitable glidant is silica.

Examples of sweeteners are acesulfame K, sucralose, alitame, aspartame, saccharin sodium, dipotassium glycyrrhizinate, stevia and thaumatin.

Flavouring agents can be natural or synthetic materials, such as orange, spearmint, strawberry and cream flavour and the like.

Examples of organic acids are citric acid or tartaric acid.

In a preferred embodiment the composition according to the invention is in form of a tablet, preferably a disintegrating tablet. This tablet may have a film-coated surface.

In a particularly preferred embodiment the composition according to the invention is in form of an orally disintegrating composition, e.g. an orally disintegrating table. Such compositions, e.g. tablets, usually disintegrate in the oral cavity within 3 minutes, preferably within 1 minute.

The invention also relates to a tablet which is obtainable by compressing a mixture of
(a) the units which comprise at least one active ingredient and at least one coating,
(b) the protective granules which comprise
   (i) binder or filler selected from lactose, lactose hydrate, starch, cellulose, sugar alcohol, calcium hydrogen phosphate, and mixtures thereof, and
   (ii) additive selected from polyethylene glycol, poloxamer, sugar ester, glycerol fatty acid mono ester, and mixtures thereof, and
(c) optionally at least one tableting excipient, with the proviso that the active ingredient is not a benzimidazole compound.

Further, the invention also relates to the above described protective granules which comprise
(i) binder or filler selected from lactose, lactose hydrate, starch, cellulose, sugar alcohol, calcium hydrogen phosphate, and mixtures thereof, and
(ii) additive selected from polyethylene glycol, poloxamer, sugar ester, glycerol fatty acid mono ester and mixtures thereof.

Optionally, the protective granules may also include a water soluble polymer selected from povidone, methyl cellulose, hydroxypropyl methylcellulose, hydroxypropyl cellulose, hydroxyethyl cellulose, and mixtures thereof.

These granules are preferably prepared by a process, wherein the binder or filler and the additive are mixed, and the obtained mixture is granulated in a common granulating equipment, such as high shear mixer or a fluid bed granulator or in an extruder. It is preferred that the granulating step is carried out as a wet granulation using water as granulation liquid. In a preferred embodiment the granulation liquid contains water and at least one of binder or filler, additive and optional water soluble polymer.

The composition according to the invention is preferably prepared by a process, wherein
(a) the protective granules are prepared as above,
(b) the protective granules are mixed with the units and the optional tableting excipient, and
(c) optionally the obtained mixture is compressed.

In preferred embodiment the mixture is compressed in step (c) to give in particular tablets.

Finally, the invention also relates to the use of the protective granules according to the invention for reducing the damages to coated units containing at least one active ingredient upon compression, preferably upon compression to tablets, with the proviso that the active ingredient is not a benzimidazole compound.

It is preferred that the units employed in this use comprise an controlled-release coating. As has been mentioned above, the protective granules are surprisingly capable of substantially maintaining the integrity of controlled-release coatings of units such as pellets when these are subjected to compression forces occurring during a compaction process.

The invention is in the following further illustrated by reference to examples.

### Examples

### Example 1: Hydromorphone-HCl containing pellets with protective granules of MCC and PEG

In a first step, pellets made of inert cores with a hydromorphone-HCl containing coating, a separating coating, a sustained-release coating and an overcoating applied thereon were produced in a fluid-bed coater. The average particle size of these pellets was about 600 µm. The composition of these pellets is given below in Table 1.

Secondly, a protective granulate consisting of microcrystalline cellulose (MCC) and polyethylene glycol (PEG), namely PEG 6000, was prepared. For this purpose the MCC and PEG 6000 were mixed in a turbula mixer. The mixture was introduced in a twin-screw extruder, wetted with a sufficient amount of water via a filler plug and pressed through an aperture of 0.8 mm. The obtained extrudate was broken in a spheronizer for a few seconds. Subsequently, the resulting granules were dried using a fluid-bed drier. The composition of the granulate is also given below in Table 1.

Further, the prepared protective granulate was mixed with the sustained-release pellets and additional tableting excipients to give a tableting mixture. The type and amount of tableting excipients is also given below in Table 1.

Finally, the tableting mixture was compressed on a laboratory rotary tablet press to oblong shaped disintegrating tablets with a dimension of 18x8 mm and a weight of about 600 mg.

**Table 1**

| ***Composition of hydromorphone-HCl tablets:*** |
|---|
| Sustained-release coated pellets |
| Protective granules consisting of microcrystalline cellulose and PEG 6000 |
| Tableting excipients |

**Composition of the sustained-release pellets:**

| **Example No.** | **1** |
|---|---|
| | mg / DF |
| **Active Core** | |
| Hydromorphone-HCl | 24.0 |
| MCC pellets | 91.2 |
| Hypromellose | 4.8 |
| | |

| **Separating coating** | |
|---|---|
| Hypromellose | 2.4 |
| Talc | 1.2 |
| | |

| **Sustained-release coating** | |
|---|---|
| Kollicoat SR | 20.4 |
| Talc | 4.9 |
| Propylene glycol | 4.1 |
| | |

| **Overcoating** | |
|---|---|
| Hypromellose | 10 |
| MCC 105 | 30 |

**Composition of protective granules**

| **Example No.** | **1** |
|---|---|
| MCC 101 | 184.1 |
| PEG 6000 | 46.0 |

**Type and amount of tableting excipients**

| **Example No.** | **1** |
|---|---|
| MMC 101 | 149.0 |
| Talc | 9.0 |
| Magnesiumstearate | 9.0 |
| Crospovidone | 10.0 |
| | |
| **Total weight of tablet (mg)** | **600.0** |

### Example 2 and 3:

Example 2 (comparative) differs from example 1 only in that no granulate of MCC and PEG 6000, but only a mixture of them prepared in a turbula mixer was used. As is apparent from Table 2, the dissolution rate of the obtained hydromorphone-HCl tablets was significantly changed in case of example 2 (comparative) indicating a substantial damage to the sustained-release coating of the pellets upon their compression to the tablets. In contrast to this, the tablets of example 1 showed only slight changes in the dissolution profile.

Moreover, in example 3 (comparative) the same sustained-release pellets as were used in examples 1 and example 2 were checked for their dissolution rate. These pellets were not compressed and therefore had an undamaged sustained-release coating. It is very surprising that the tablets of example 1 showed, relative to the uncompressed pellets, only a slightly changed dissolution profile even though they had been prepared by actually subjecting the pellets to the damaging compression forces.

**Table 2**

| **Example No.** | **1** | **2** | **3** |
|---|---|---|---|
| | | | |
| | **Granulate of MCC and PEG** | **Mixture of MCC and PEG** | |
| | | | |
| Sustained-release pellets | 193.0 | 193.0 | 193,0 |
| | | | |
| MCC | 184.1 | 184.1 | |
| PEG 6000 | 46.0 | 46.0 | |
| | | | |
| MCC 101 | 149.0 | | |
| Talc | 9.0 | 9.0 | |
| Magnesiumstearate | 9.0 | 9.0 | |
| Crospovidone | 10.0 | 10.0 | |
| | | | |
| | | | |
| **Total weight (g)** | **600.0** | **600.0** | **193.0** |

| Dissolution profile [mean value, n=6]* | | | |
|---|---|---|---|
| 1h: | 13.3% | 20.0% | 5.5% |
| 2h: | 24.4% | 30.4% | 18.6% |
| 3h: | 36.2% | 44.5% | 33.2% |
| 4h: | 49.1% | 54.9% | 47.4% |
| 5h: | 62.3% | 64.0% | 60.7% |
| 6h: | 71.8% | 73.8% | 71.1% |
| 7h: | 78.1% | 79.7% | 79.3% |
| 8h: | 82.7% | 83.1% | 86.6% |
| | | | |
| **Uniformity of mass**** | **1.9%** | **9.2%** | |
| **Content uniformity**** | **2.4%** | **8.9%** | |

| | | | |
|---|---|---|---|
| * The dissolution test was performed using a paddle apparatus (app. No 2 according to USP) with a rotating speed of 100rpm. As dissolution medium 900 ml of phosphate buffer pH 6.4 was used. The amount of active ingredient was evaluated by HPLC. ** Determined according to Ph. Eur. 5.0. The given values are relative standard deviations. | | | |

## Claims

1. Pharmaceutical multiple-unit composition comprising
(a) units which comprise at least one active ingredient and at least one coating,
(b) protective granules which comprise
(i) binder or filler selected from lactose, lactose hydrate, starch, cellulose, sugar alcohol, calcium hydrogen phosphate, and mixtures thereof, and
(ii) additive selected from polyethylene glycol, poloxamer, sugar ester, glycerol fatty acid mono ester, and mixtures thereof, and
(c) optionally at least one tableting excipient,
with the proviso that the active ingredient is not a benzimidazole compound.

2. Composition according to claim 1, wherein the units are beads, particles, granules or pellets and preferably are pellets.

3. Composition according to claim 1 or 2, wherein the active ingredient is selected from the group of analgesics, antidepressants and non-steroid antiinflammatories.

4. Composition according to claim 3, wherein the active ingredient is hydromorphone or amitryptiline or salts thereof.

5. Composition according to any one of claims 1 to 4, wherein the coating is selected from an enteric coating, a controlled-release coating, a taste-masking coating, a separating coating, an intermediate coating, an overcoating, an active ingredient-containing coating or combinations thereof.

6. Composition according to any one of claims 1 to 5, wherein the units comprise an active ingredient-containing core.

7. Composition according to any one of claims 1 to 6, wherein the units comprise at least one controlled-release coating.

8. Composition according to claim 7, wherein the units comprise an active ingredient-containing coating, a controlled-release coating and an overcoating.

9. Composition according to claim 7, wherein the units comprise an active ingredient-containing core, a controlled-release coating and an overcoating.

10. Composition according to any one of claims 5 to 9, wherein the active ingredient-containing coating or the active ingredient containing core comprises hydroxypropyl methylcellulose.

11. Composition according to any one of claims 5 to 10, wherein the controlled-release coating comprises polyvinyl acetate polymer.

12. Composition according to any one of claims 5 to 11, wherein the overcoating comprises water soluble binder selected from methyl cellulose, hydroxypropyl methylcellulose, hydroxypropyl cellulose, hydroxyethyl cellulose, polyvinyl pyrrolidone, polyethylene glycol and mixtures thereof.

13. Composition according to any one of claims 5 to 12, wherein the overcoating comprises water insoluble substance selected from microcrystalline cellulose, starch and mixtures thereof.

14. Composition according to claims 12 or 13, wherein the overcoating comprises hydroxypropyl methylcellulose and microcrystalline cellulose.

15. Composition according to any one of claims 1 to 14, wherein the binder (i) is microcrystalline cellulose.

16. Composition according to any one of claims 1 to 15, wherein the additive (ii) is polyethylene glycol, and preferably polyethylene glycol of the formula H(OCH₂-CH₂)ₙOH wherein n is in the range of 100 to 250 and in particular 150 to 210.

17. Composition according to claim 15 or 16, wherein the protective granules consist of microcrystalline cellulose and polyethylene glycol(s).

18. Composition according to any one of claims 1 to 17, wherein the tableting excipient (c) is at least one disintegrant.

19. Composition according to claim 18, wherein the disintegrant is cross-linked polyvinyl pyrrolidone.

20. Composition according to any one of claims 1 to 19, wherein the composition comprises 10 to 50 % by weight of the units.

21. Composition according to any one of claims 1 to 20, wherein the composition comprises 10 to 45 % by weight of the protective granules.

22. Composition according to any one of claims 1 to 21, wherein the protective granules comprise 50 to 95 and preferably 70 to 85 % by weight of binder or filler (i).

23. Composition according to any one of claims 1 to 22, wherein the protective granules comprise 5 to 50 and preferably 15 to 30 % by weight of additive (ii).

24. Composition according to any one of claims 1 to 23, wherein the protective granules comprise binder or filler (i) and additive (ii) in a weight ratio in the range of 1:1 to 20:1 and preferably 2:1 to 5:1.

25. Composition according to any one of claims 1 to 23, wherein the composition comprises units and protective granules in a weight ratio in the range of 0.4:1 to 5:1 and preferably 0.5:1 to 3:1.

26. Composition according to any one of claims 1 to 25, wherein the average particle size of the protective granules does not differ by more than 50 % from the average particle size of the units.

27. Composition according to any one of claims 1 to 26, which is an orally disintegrating composition.

28. Composition according to any one of claims 1 to 27, which is in form of a tablet or a film-coated tablet.

29. Tablet which is obtainable by compressing a mixture of
(a) units which comprise at least one active ingredient and at least one coating,
(b) protective granules which comprise
(i) binder or filler selected from lactose, lactose hydrate, starch, cellulose, sugar alcohol, calcium hydrogen phosphate, and mixtures thereof, and
(ii) additive selected from polyethylene glycol, poloxamer, sugar ester, glycerol fatty acid mono esters, and mixtures thereof, and
(c) optionally at least one tableting excipient,
with the proviso that the active ingredient is not a benzimidazole compound.

30. Protective granules which comprise
(i) binder or filler selected from lactose, lactose hydrate, starch, cellulose, sugar alcohol, calcium hydrogen phosphate, and mixtures thereof, and
(ii) additive selected from polyethylene glycol, poloxamer, sugar ester, glycerol fatty acid mono ester, and mixtures thereof.

31. Process for preparing the protective granules according to claim 30, wherein the binder or filler and the additive are mixed, and the obtained mixture is granulated.

32. Process for preparing the composition according to any one of claims 1 to 28, wherein
(a) the protective granules are prepared according to the process of claim 31,
(b) the protective granules are mixed with the units and the tableting excipient, and
(c) optionally the obtained mixture is compressed.

33. Use of the protective granules according to claim 30 for reducing the damages to coated units containing at least one active ingredient upon compression, preferably upon compression to tablets, with the proviso that the active ingredient is not a benzimidazole compound.

34. Use according to claim 33, wherein the units comprise a controlled-release coating.
